# EUROPEAN PATENT APPLICATION

(11) **EP 3 363 429 A1**
(43) Date of publication of application: **22.08.2018**
(21) Application number: 17156874.4
(22) Date of filing: 20.02.2017
(51) Int. Cl.: A61K 9/16, A61K 9/50

(54) **PROCESS FOR THE PRODUCTION OF COATED PARTICLES**

(71) Applicant: DSM IP Assets B.V., 6411 TE Heerlen (NL)
(72) Inventor: The designation of the inventor has not yet been filed
(74) Representative: Kurt, Manfred

(57) **Abstract**

The present patent application relates to a process for the production of specific coated particles, wherein the coating (system) comprises at least one wax and/or at least one fat and wherein specific process parameters are applied.

## Description

The present patent application relates to a process for the production of specific coated particles, wherein the coating (system) comprises at least one wax and/or at least one fat and wherein specific process parameters are applied.

Particles which are used to be compacted (compressed) into tablets usually contain one or more active ingredient, which are essential for the tablet (for need of the consumers).
Examples for such active ingredients are vitamins, polyunsaturated fatty acids, carotenoids, oils, minerals, plants extracts or any other commonly used active ingredient. These active ingredients are first formulated into particles, which are then used for (for example) producing compressed (compacted) tablets or they are used in premixes which are then used for further formulations (such as compressed or compacted tablets).

A major problem and disadvantage in the use of such particles is that when pressure (usually more than 5kN) is applied on such particles (which comprise the essential ingredients for the tablet), then some of the essential ingredients are usually squeezed out of the particle, degraded within days and lost for further formulation. Furthermore, a smell issue or a discoloration issue can also arise because of this "squeezing-out"-phenomenon, depending on the active ingredient. This well-known effect is called "initial loss".
To assure that in the final product (for example a compressed or compacted tablet) the correct e.g. desired) amount of the essential ingredient is present, an overdosage of the essential ingredient is usually used nowadays. But overdosage is not an ideal solution of this problem.

It was found that when specific coated particles are used, these above mentioned problems are solved.

These coated particles (CP) comprise
(a) 40 wt-% - 95 wt-%, based on the total weight of the coated particle, of a core, comprising at least one active ingredient and
(b) 5 wt-% - 60 wt-%, based on the total weight of the coated particle, of a coating system wherein said coating system comprises at least one wax and/or at least one fat.

The term "coated particle" in the context always refers to a core which is coated by a coating system. Usually the coating covers the whole particle, but it can also have some "defects" where no coating is present (but more than 90% of the surface of the particle is coated).
The term "core" in the context of the present invention always refers to a particle, which is produced by any commonly known and used technology (such as spray drying, spray cooling, spray granulation, spray chilling etc.) and which contains at least one active ingredient.
The coated particles are usually of such a size that tablets can be compacted.
A suitable size of the coated particles is between 50 - 1000µm (preferably 100 - 800 µm); the size is defined by the diameter of the longest dimension of the particle and measured by commonly known method (like light scattering).
All particle sizes of the solid particles according to the present invention are determined by laser diffraction technique using a "Mastersizer 3000" of Malvern Instruments Ltd., UK. Further information on this particle size characterization method can e.g. be found in "Basic principles of particle size analytics", Dr. Alan Rawle, Malvern Instruments Limited, Enigma Business Part, Grovewood Road, Malvern, Worcestershire, WR14 1XZ, UK and the "Manual of Malvern particle size analyzer". Particular reference is made to the user manual number MAN 0096, Issue 1.0, Nov. 1994. If nothing else is stated all particle sizes referring to the coarse particles of the solid particles according to the present invention are Dv90 values (volume diameter, 90% of the population resides below this point, and 10% resides above this point) determined by laser diffraction. The particle size can be determined in the dry form, i.e. as powder or in suspension. Preferably, the particle size of the solid particles according to the present invention is determined as powder.

The distribution of the particle size is also no essential feature of the present invention.

The shape of the core as well as of the coated particles is also not an essential feature of the present invention. The shape can be sphere-like or any other form (also mixtures of shapes). Usually and preferably the particles are sphere-like.

The coating system used in the coating process according to the present invention comprises at least one wax and/or at least one fat.
Waxes in the context of the present invention are organic compounds that characteristically consist of long alkyl chains. Natural waxes (plant, animal) are typically esters of fatty acids and long chain alcohols. Synthetic waxes are long-chain hydrocarbons lacking functional groups.

Fats, which are used for the embodiments of the present invention, consist of a wide group of compounds that are generally soluble in organic solvents and largely insoluble in water. Hydrogenated fats (or saturated fats) in the context of the present invention are generally triesters of glycerol and fatty acids. Fatty acids are chains of carbon and hydrogen atoms, with a carboxylic acid group at one end. Such fats can have natural or synthetic origin. It is possible to hydrogenate a (poly)unsaturated fat to obtain a hydrogenated (saturated) fat.

Especially suitable waxes and fats have a dropping point of from 30 to 85°C, preferably 40 to 70°C.

The dropping point of a material is that temperature (in °C) when the material begins to melt under standardized conditions. The material is heated so long until it changes the state of matter from solid to liquid. The dropping point is the temperature when the first dropping is released from the material. The determination of the dropping point (Tropfpunkt) is carried out as described in the standard norm DIN ISO 2176.

Preferred examples of waxes and fats suitable for the present invention are glycerine monostearate, carnauba wax, candelilla wax, soya fat, sugarcane wax, palmitic acid, stearic acid, (fully) hydrogenated cottonseed oil, (fully) hydrogenated palm oil and (fully) hydrogenated rapeseed oil. These compounds can be used as such or as mixtures. Preferred are carnauba wax, candelilla wax, sugarcane wax and (fully) hydrogenated palm oil.

The coating system of the coated particles can also comprise further ingredients, which are non-essential for the quality of the coated particles. Such ingredients can be dyes, flavours, or any other ingredient.
The core of the coated particles comprises at least one active ingredient, which is needed. (for example when the coated particle is used in a compressed tablet). That active ingredient (or mixture of active ingredients) can be any kind of active ingredient. The ingredients can be oil-soluble or water-soluble.
Suitable ingredients are for example any vitamins, carotenoids, polyunsaturated fatty acids (PUFA), minerals, plant extracts or any other active ingredient.
Suitable ingredients are fat-soluble vitamins, such a vitamin A, D, E, and K (as well as derivatives thereof); water-soluble vitamins such as B-vitamins and vitamin C; and carotenoids such as α-carotene, β-carotene, 8'-apo-ββ-carotenal, 8'-apo-β-carotenoic acid esters, canthaxanthin, curcumin, astaxanthin, lycopene, lutein, zeaxanthin and crocetin.

Preferred active ingredients in the context of the present invention are fat-soluble vitamins. These are vitamins A, D, E, and K (as well as derivatives of any of these vitamins). Especially preferred is vitamin A and/or its derivatives.

Also preferred cores are such which comprises (as well as consists of)
(i) 20 weight-% (wt-%) - 75 wt-% (preferably 20 wt-% - 70 wt-%), based on the total weight of core, of least one fat soluble vitamin chosen form the group consisting of A, D, E, and K (as well as derivatives thereof; preferred is vitamin A and/or its derivatives (such as vitamin A acetate and vitamin A palmitate)), and
(ii) 20 wt-% - 70 wt-% (preferably 25 wt-% - 65 wt-%) , based on the total weight of core, of at least one emulsifier, which does not derive from an amimal source (preferably chosen from the group consisting of modified (food) starches, polysaccharide gums and plant proteins, and
(iii) 5 wt-% - 55 wt- %, (preferably 15 wt-% - 45 wt-%), based on the total weight of the core, of at least one non-reducing sugar, which is preferably sucrose and/or trehalose, most preferred is trehalose, and
(iv) optionally up to 15 wt-%, based on the total weight of the core, of at least one auxiliary agent chosen from the group consisting of antioxidants (such as ascorbic acid or salts thereof, tocopherol (synthetic or natural)), butylated hydroxytoluene (BHT), ascorbyl palmitate, butylated hydroxyanisole (BHA), propyl gallate, tert. butyl hydroxyquinoline, ethoxyquin and/or ascorbic acid esters of a fatty acid); stabilisers (such as gel-forming agents as xanthan gum, gellan gum); humectants (such as glycerine, sorbitol, polyethylene glycol); dyes; fragrances; fillers and buffers.

It was found that these coated particles do have excellent functional properties when produced using the specific coating process wherein well-defined parameters are applied.

It was found that the choice of the process parameter used in the coating process are essential to get an improved coated particle.

The coating process is carried out in such a way that the cores (solid particles, which need to be coated) are brought in contact with the coating material (can be a single compound or a mixture of compounds), which is in a state that it can coat the cores. The coating process is usually carried out by using commercially available coating devices. Such devices can be bought from companies as GEA Pharma Systems Technology AG, Glatt GmbH, etc.
Commercially available coating devices can be used and the specific process parameters can applied when using them.

Coating process are known in the prior art. Commonly known and used are for example among others spray chilling, spray coating, fluidized bed spray granulation and film coating.

It was found that spray coating with specific process parameter is producing coated particles with excellent properties.
The essential process parameter is
(i) the target product temperature.

Additionally there are more specific and well-defined process parameters which, when applied, can further improve the coated particles.

These process parameters are
(ii) the inlet air temperature
(iii) the atomising air temperature.
(iv) the inlet air specific humidity
(v) the inlet air rate
(vi) the atomising air pressure

In general there are 4 steps in a coating process:
(I) the loading period (= period (I)) followed by
(II) the pre-heating period (= period (II)) followed by
(III) the liquid addition period (= period (III)) followed by
(IV) the cooling period (= period (IV)).

Therefore the present invention relates to a coating process (P) for the production of coated particles (which have been defined in more details above), which comprise
(a) 40 wt-% - 95 wt-%, based on the total weight of the coated particle, of a core, comprising at least one active ingredient and
(b) 5 wt-% - 60 wt-%, based on the total weight of the coated particle, of a coating system wherein said coating system comprises at least one wax and/or at least one fat,
characterised in that the coating process is carried in such a way that
(i) the target product temperature of 30 - 45°C is achieved.

All preferences in regard to the coated particles as well as the core are also applicable for the process (P) as well as the further more preferred processes, which are defined in the following.

All the process parameters are well-known parameters and also their way of measuring and determining them.

The target product temperature is measured in °C. The target product temperature is usually 30 - 45°C. It is measured with a probe (which is brought in contact with the coated particles) inside the coating device.

Furthermore the coating process according to the present invention is can be carried out by using well defined

These process parameters are
(ii) the inlet air specific humidity;
(iii) the inlet air speed;
(iv) the atomising air pressure
(v) the inlet air temperature
(vi) the atomising air temperature

When not specified otherwise, the parameters specified below are the same for all periods of the coating process.

The inlet air specific humidity is measured in gram water per kilogram air. The inlet air specific humidity is 0 - 15, preferably 3 - 10. It is measured at the entry (into the coating device) of the inlet air.

Therefore the present invention also relates to a coating process (P1), which is coating process (P), wherein the inlet air specific humidity is 0 - 15 gram water per kilogram air, preferably 3 - 10 gram water per kilogram air.

The inlet air rate is measured in cubic meter air per hour (m³/h). The inlet air speed is given in m/s and it is defined as inlet air rate per surface of bottom of the device. The inlet air speed is usually 0.5 - 3.0 m/s. (preferably 0.75 - 2.5 m/s).

Therefore the present invention also relates to a coating process (P2), which is coating process (P) or (P1), wherein the inlet air speed is 0.5 - 3.0 m/s. (preferably 0.75 - 2.5 m/s).

The atomising air pressure is measured in bar. The atomising air pressure is measured at the entry of the spraying nozzle. The atomising air pressure is usually 0.25 - 8 bar, preferably 0.5 - 6 bar.

Therefore the present invention also relates to a coating process (P3), which is coating process (P), (P1) or (P2), wherein the atomising air pressure is 0.25 - 8 bar (preferably 0.5 - 6 bar).

The inlet air temperature is given in °C. It is the temperature which measured at the point of coating device wherein the air enters the device. The inlet air temperature for period (II) and (III) is between 35 - 50 °C.
The given temperature range is valid and is applied for period (II) and period (III). The inlet air temperature for the cooling phase (phase (IV)) is between is usually below 35°C, preferably between 20 - 30 °C. Ideally it is room temperature.

Therefore the present invention also relates to a coating process (P4), which is coating process (P), (P1), (P2) or (P3), wherein the inlet air temperature for period (II) and (III) is 35 - 50°C.
Therefore the present invention also relates to a coating process (P5), which is coating process (P), (P1), (P2), (P3) or (P4), wherein the inlet air temperature for period (IV) is 20 - 30 °C (preferably it is room temperature).

The atomising air temperature is the temperature, which is used at the nozzle to atomize the coating system.
The atomising air temperature is usually kept constant during the whole coating process (in all 4 periods). The atomising air temperature usually is between 90 - 130 °C, preferably 100 - 120°C.

Therefore the present invention also relates to a coating process (P6), which is coating process (P), (P1), (P2), (P3), (P4) or (P5), wherein the atomising air temperature is between 90-130 °C (preferably 100 - 120°C).

As an alternative it is also possible to heat the nozzle (instead of heating the air) or both.

In view of the device which is used to carry out the coating process it is possible to use
(A) a top spray device
(B) a tangential spray device or
(C) a bottom spray device or
(D) Wurster device.

All these devices are available commercially from various suppliers as mentioned above already.
The invention is illustrated by the following Examples. All temperatures are given in °C and all parts and percentages are related to the weight.

### Examples

### Example 1

A total of 300g of particle containing vitamin A acetate having a particle size distribution between 150µm and 600µm were coated on a DMR WFP-Mini fluidized bed coating using top spray set up. The cores were introduced into the reactor at room temperature and conditioned at 35°C before spraying the coating material. Palm oil full hydrogenated (128.6g) was molten beforehand at 95°C and sprayed at a rate of about 3 g.min⁻¹. An atomizing air pressure of 1 bar and an atomizing air temperature of 100 to 120°C were used for the entire process. The product temperature was kept between 40 to 42°C. The entire process was concluded after approximately 50 min. Then the product was cooled down to RT in the reactor and the collected product was sieved to isolate three main fractions, i.e. below 160 µm, between 160 to 500 µm and higher than 500 µm in size.
Product collected 420.4g.
Yield below 500 µm : 96.53%.

| Sieving characteristics | Amount of product [%] |
|---|---|
| <160 µm | 0.03 |
| 160-500µm | 96.5 |
| >500µm | 1.6 |
| Total | 98.1 |
| Theory | 100 |

### Example 2

A total of 300g of particles containing vitamin A acetate having a particle size distribution between 150µm and 600µm were coated on a DMR WFP-Mini fluidized bed coating using top spray set up. The cores were introduced into the reactor at room temperature and conditioned at 35°C before spraying the coating material. Palm oil full hydrogenated (128.6g) was molten beforehand at 95°C and sprayed at a rate of about 4 to 5 g.min⁻¹. An atomizing air pressure of 1 bar and an atomizing air temperature of 100 to 120°C were used for the entire process. The product temperature was kept between 40 to 42°C. The entire process was concluded after approximately 50 min. Then the product was cooled down to RT in the reactor and the collected product was sieved to isolate three main fractions, i.e. below 160 µm, between 160 to 500 µm and higher than 500 µm in size.
Product collected 424.7g - Yield below 500 µm : 86.66%.

| Sieving characteristics | Amount of product [%] |
|---|---|
| <160 µm | 0.05 |
| 160-500µm | 86.6 |
| >500µm | 13.5 |
| Total | 99.1 |
| Theory | 100 |

### Example 3 (tangential)

About 900g of cores of particles containing vitamin A acetate having a particle size distribution between 150µm and 600µm were coated on a GEA-Niro-AEROMATIC MP1 fluidized bed coating using tangential spray set up. About 385.7g of palm oil FH was molten. The cores were introduced into the reactor at room temperature and afterward, warmed up at 38-39°C. The process was then started by spraying the molten palm oil FH on the core with an increasing spraying rate from 7 to 12 g.min⁻¹. An atomizing air pressure of 1 bar and an atomizing air temperature of 100 to 120°C were used for the entire process. The product temperature was strictly controlled and kept between 39 and 40°C for the entire spraying process. The product was cooled down to room temperature into the reactor. The collected free flowing product was sieved to isolate three main fractions.
Total product collected 1256.7g.
Yield below 500 µm: 98.8%

| Sieving characteristics | Amount of product [%] |
|---|---|
| <160 µm | 0.02 |
| 160-500µm | 98.8 |
| >500µm | 1.2 |
| Total | 100.0 |

### Example 4 (Wurster)

About 700g of particles containing vitamin A acetate having a particle size distribution between 150µm and 600µm were coated on a GEA-Niro-AEROMATIC MP1 fluidized bed coating using Wurster set up. About 300.0g of palm oil FH was molten. The cores were introduced into the reactor at room temperature and afterward, warmed up at 38-39°C. The process was then started by spraying the molten palm oil FH on the core with an increasing spraying rate of 3g.min⁻¹. An atomizing air pressure of 1.5 bar and an atomizing air temperature of 100 to 120°C were used for the entire process. The inlet temperature was decreased over process from 43 to 38°C. The product was cooled down to room temperature into the reactor. The collected free flowing product was sieved to isolate three main fractions.
Total product collected 978.8g.
Yield below 500 µm: 98.8%

| Sieving characteristics | Amount of product [%] |
|---|---|
| <160 µm | 0.1 |
| 160-500µm | 93.7 |
| >500µm | 6.2 |
| Total | 100.0 |

### Example 5 (Top)

About 1250g of particles containing vitamin A acetate having a particle size distribution between 150µm and 600µm were coated on a GEA-Niro-AEROMATIC MP1 fluidized bed coating using top spray set up. About 536.0g of palm oil FH was molten. The cores were introduced into the reactor at room temperature and warmed up at 42°C. The process was then started by spraying the molten palm oil FH on the core with a spraying rate of 5 g.min⁻¹ up to 6 g.min⁻¹. An atomizing air pressure of 1 bar and an atomizing air temperature of 100 to 120°C were used for the entire process. The inlet temperature kept constant at 40°C and the product temperature monitored. The product was cooled down to room temperature into the reactor. The collected free flowing product was sieved to isolate three main fractions.
Total product collected 1640g.
Yield below 500 µm: 98.6%

| Sieving characteristics | Amount of product [%] |
|---|---|
| <160 µm | <0.1 |
| 160-500µm | 98.6 |
| >500µm | 1.4 |
| Total | 100.0 |

## Claims

1. A coating process for the production of coated particles (which have been defined in more details above), which comprise
(a) 40 wt-% - 95 wt-%, based on the total weight of the coated particle, of a core, comprising at least one active ingredient and
(b) 5 wt-% - 60 wt-%, based on the total weight of the coated particle, of a coating system wherein said coating system comprises at least one wax and/or at least one fat,
**characterised in that** the coating process is carried in such a way that
(i) the target product temperature of 30 - 45°C is achieved.

2. Coating process according to claim 1, wherein the inlet air specific humidity is 0 - 15 gram water per kilogram air, preferably 3 - 10 gram water per kilogram air.

3. Coating process according to any of the preceding claims, wherein the inlet air speed is 0.5 - 3.0 m/s. (preferably 0.75 - 2.5 m/s).

4. Coating process according to any of the preceding claims, wherein the atomising air pressure is 0.25 - 8 bar (preferably 0.5 - 6 bar).

5. Coating process according to any of the preceding claims, wherein the inlet air temperature for period (II) and (III) is 35 - 50°C.

6. Coating process according to any of the preceding claims 1 - 4, wherein wherein the inlet air temperature for period (IV) is 20 - 30 °C (preferably room temperature).

7. Coating process according to any of the preceding claims, wherein the atomising air temperature is between 90 - 130 °C (preferably 100 - 120°C).

8. Coating process according to any of the preceding claims, wherein the coating process is carried out in a top spray device.

9. Coating process according to any of the preceding claims 1 - 7, wherein the coating process is carried out in a tangential spray device.

10. Coating process according to any of the preceding claims, wherein the coating process is carried out in a bottom spray device.

11. Coating process according to any of the preceding claims, wherein the coating process is carried out in a Wurster device.
